Europäisches Patentamt

European Patent Office

Office européen des brevets

(19)

(11) **EP 0 748 193 B1**

(12) **EUROPEAN PATENT SPECIFICATION**

(45) Date of publication and mention
of the grant of the patent:
**05.12.2001 Bulletin 2001/49**

(51) Int Cl.⁷: **A61F 2/34**

(21) Application number: **95909061.4**

(86) International application number:
**PCT/GB95/00403**

(22) Date of filing: **27.02.1995**

(87) International publication number:
**WO 95/23566 (08.09.1995 Gazette 1995/38)**

(54) **ACETABULAR CUP**

HÜFTGELENKPFANNE

CAVITE COTYLO DE

(84) Designated Contracting States:
**AT BE CH DE DK ES FR GB IE LI NL PT SE**

(30) Priority: **03.03.1994 GB 9404077**

(43) Date of publication of application:
**18.12.1996 Bulletin 1996/51**

(73) Proprietor: **THE UNIVERSITY OF LEEDS**
**Leeds, LS2 9JT (GB)**

(72) Inventor: **FISHER, John**
**Department of Mechanical Engineering**
**Leeds West Yorkshire LS2 9JT (GB)**

(74) Representative:
**Tunstall, Christopher Stephen et al**
**Harrison Goddard Foote,**
**Fountain Precinct,**
**Leopold Street**
**Sheffield S1 2QD (GB)**

(56) References cited:
**EP-A- 0 226 762       EP-A- 0 302 850**
**WO-A-86/01394      DE-A- 2 742 464**
**DE-A- 3 630 276      DE-A- 3 840 468**
**FR-A- 2 134 170      FR-A- 2 315 903**
**FR-A- 2 413 078**

EP 0 748 193 B1

## Description

This invention relates to an acetabular cup and to a prosthetic hip joint consisting of the acetabular cup and a femoral component. In particular, the invention relates to such a joint having improved lubrication qualities.

## Background to the invention

Over half a million artificial hip joints are implanted in patients every year. These are all ball and socket joints with a spherical femoral head and a hemispherical acetabular cup or socket. Over 95% of these comprise a hard metal or ceramic head, with an ultra-high molecular weight polyethylene cup. Other types include metal on metal and ceramic on ceramic and, more recently, soft or compliant bearing surfaces, termed "cushion form bearings". All these joints have a primarily spherical geometry with the radius of curvature of the head $R_1$ being smaller than the radius of curvature of the socket $R_2$. This allows the joint to be assembled and free to move. Typically, the radial clearance $R_2 - R_1$ is between 0.05 and 0.5mm. The smaller radial clearances are used for hard on hard prostheses, such as metal on metal and ceramic on ceramic, with larger clearances being used for cushion form bearings. For commonly used polyethylene $R_1 - R_2$ is between 0.01 and 0.03mm. Typically, when the two spheres come into contact at a point the materials then deform elastically with the contact area increasing as the load and contact stress increase. The radial clearance and the elasticity of the materials determine the contact area, stress and lubricating film thickness, all of which determine the wear of the materials.

In hard on hard and cushion form bearings, there are real concerns about high levels of friction at start up after periods of high constant load when the lubricating film has been squeezed out from between the two joint components. It has been proposed in metal on metal joints to form a dimple or local discontinuity in the surface of the cup at the pole, in an attempt to trap the fluid and reduce friction. It is not clear whether this works, but it has been established that the dimple produces discontinuities in the stress field.

EP 0 302 850 discloses an acetabular cup having a concave bearing socket adapted to receive the head of the femoral component of a hip prosthesis. The radius of curvature of the socket increases from the lip of the socket.

It is an object of this invention to provide a prosthetic hip joint with improved lubrication and reduced wear.

## Summary of the invention

According to the present invention, there is provided an acetabular cup having a concave bearing socket adapted to receive the head of the femoral component of a hip prosthesis, characterised in that the radius of curvature of the surface of the socket continuously and monotonically decreases from the lip of the socket to a minimum $R_2$ at a point within the socket. The acetabular cup according to the invention provides distinct advantages when used in conjunction with a standard femoral component having a substantially spherical head when $R_2$ is no greater than the radius of the spherical head of the femoral component $R_1$. When $R_2$ is equal to $R_1$, there is no single point of contact between the spherical head and the acetabular cup, but rather an enlarged contact area through which more uniform pressure is applied under load. Furthermore, since towards the lip of the socket the radius of curvature increases - and hence the amount of curvature decreases - larger space is defined between the socket and the head than would be the case with two spherical components.

When $R_1$ is greater than $R_2$, the bearing still fits together by virtue of this continuous monotonic increase in the radius of curvature as the lip of the socket is approached, but importantly a pool of lubricating fluid is trapped at the pole of the socket. Both of these arrangements contribute to more effective "squeeze film lubrication" which occurs when load is applied to the joint and a film of lubricating fluid is squeezed between the two components. They also both act to spread the applied load more effectively over the surfaces of the components, either over a large contact area towards the pole or over a contact area spaced from the distributed around the pole.

Preferably, the surface of the socket approximates that of a sphere at the point of minimum radius of curvature. In these circumstances, it is preferred that $R_2$ is equal to $R_1$. This ensures that there is a large contact area between the femoral head and the acetabular cup socket as discussed previously.

Alternatively or additionally, it is preferred that the surface of the socket coincides with that of a sphere at the intersection of the surface of the socket with a plane. In these circumstances, it is also preferred that $R_2$ be less than $R_1$ and that the radius of the sphere with which the surface of the socket coincides at the intersection of the surface of the socket with the plane be equal to $R_1$. In this way, an annular contact ring may be formed between the socket and the head which traps lubricating fluid between the two towards the point of minimum radius of curvature.

Both of the above preferred conditions may be satisfied in a single socket by arranging for the surface of the socket to be a surface of revolution about the normal to the surface at the point of minimum radius of curvature.

## Brief description of the drawings

The present invention will now be described by way of example with reference to figures 1 to 3 of the accompanying drawings in which:

Figure 1 illustrates a conventional sphere on sphere prosthetic hip joint;

Figure 2 illustrates a joint according to the invention where $R_1$ is greater than $R_2$;

Figure 3 illustrates a joint according to the present invention where $R_1$ equals $R_2$; and

Figure 4 illustrates a particular geometry of the acetabular cup.

**Detailed description**

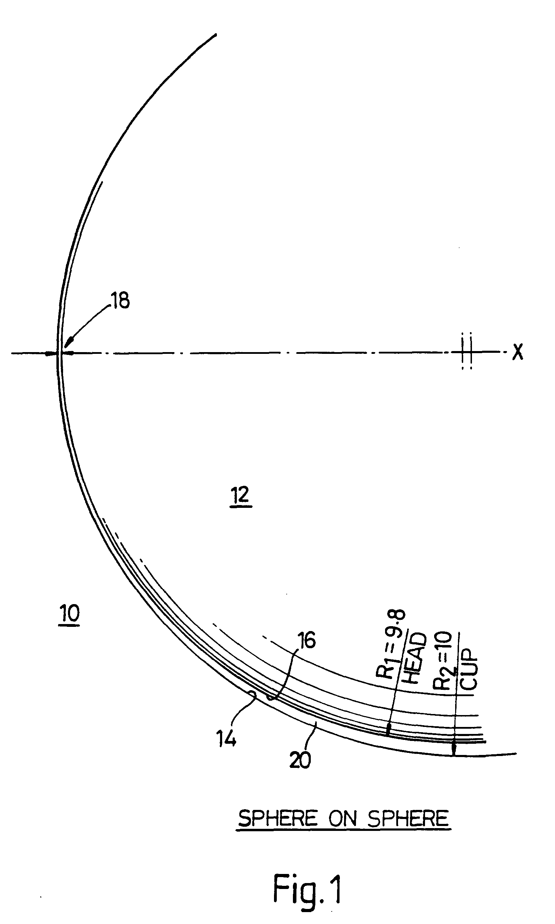

Figure 1 illustrates a conventional sphere on sphere prosthetic hip joint. The joint consists of an acetabular cup 10 and a spherical head 12 of a femoral component. The cup 10 includes a socket with a spherical concave surface 14 and the surface of the spherical head 12 is referenced 16. In the example illustrated, the radius $R_1$ of the surface 16 is 9.8mm. and the radius $R_2$ of the surface 14 is 10mm, giving a difference of 0.2mm. This difference affords a clearance 20 between the two components of the joint and lubricating fluid is able to penetrate between the two within this clearance.

When the joint is placed under load, initial contact between the two surfaces 14, 16 occurs at a point 18. As the load is increased, surface deformation will cause the contact area to spread until the elastic restoring forces are equal to the applied load.

Because the predominant movement of the natural hip joint is one of rotation about the femoral head, the two radii $R_1$ and $R_2$ are selected to be close to one another, thereby reducing to a minimum the amount of play in the joint. As a result, the squeeze film lubrication performance of this joint is relatively poor.

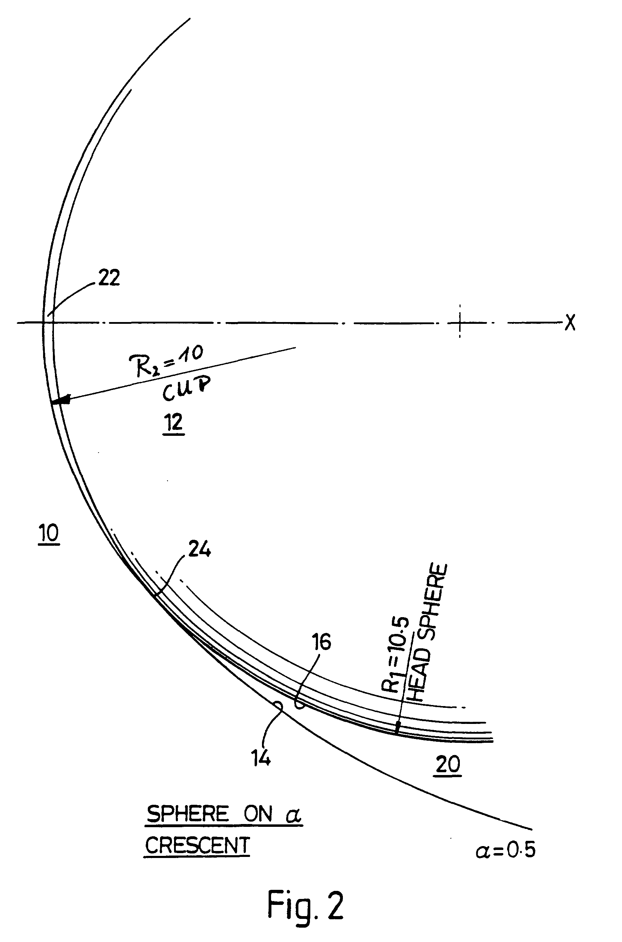

Figure 2 illustrates an embodiment of the present invention in which $R_1$ is greater than $R_2$. The joint consists of an acetabular cup 10 and the spherical head 12 of a femoral component. The surface of the spherical head is referenced 16. In this case, the surface 14 of the socket in the acetabular cup 10 is defined by an "alpharabola". The use of the alpharabola as the geometry of the socket allows the rate of change of the curvature of the surface to be varied continuously over the surface and this rate of change can be determined with a single parameter $\alpha$, once the desired minimum curvature is known. This allows the position and size of the contact area on the head 12 and cup 10 to be varied, permitting pools of lubricating fluid 22 to be trapped in the contact to assist lubrication. In addition, the need for a radial clearance between the head 12 and the cup 10 is avoided.

The alpharabola geometry is described by:

$$\alpha x^2 + y^2 + z^2 = 2R_2 x$$

$$0 < \alpha < 1$$

where:

$R_1$ is the desired minimum radius of curvature;

$x$ is a Cartesian co-ordinate representing distance from the point of minimum radius of curvature along the normal to the surface at that point; and

$y$ and $z$ are the remaining Cartesian co-ordinates.

All these geometries are symmetrical about the x axis, ie. about the normal to the surface at the point of minimum radius of curvature, and this ensures that the alpharabola surfaces are surfaces of revolution about the x axis.

In figure 2, $R_2$ is 10mm and the parameter $\alpha$ is selected to be 0.5. The radius $R_1$ of the head 12 is 10.5mm. As can be seen, a pool of fluid 22 is trapped at the pole of the alpharabola and there is an annular contact ring 24 between the two surfaces 14, 16 which serves to trap the fluid. In addition, the increasing radius of curvature of the surface 14 of the cup 10 as one approaches the rim ensures that the two surfaces 14, 16 define a relatively large clearance 20 at the rim of the cup. This facilitates the ingress of lubricating fluid.

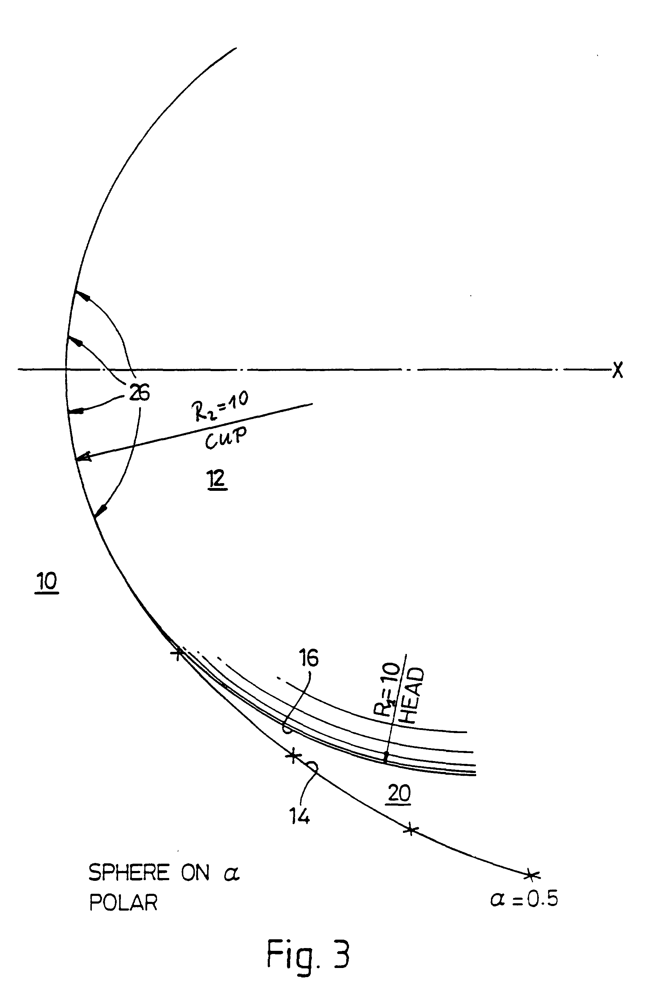

Figure 3 illustrates a joint according to the invention in which $R_1$ is equal to $R_2$ at 10mm. Again, the parameter $\alpha$ is selected to be 0.5. As can be seen, there is again a relatively large clearance 20 between the two surfaces 14, 16 towards the rim of the cup, but in place of an annular contact ring 24, there is an enlarged contact area 26. Again, this contributes to more effective squeeze film lubrication and less wear on the two components. The reduction in wear is important, since it in turn reduces the amount of debris in the joint, which debris can cause unwanted reaction in the surrounding tissue.

The parameter $\alpha$ can be selected according to the circumstances, but is necessarily less than unity. Usually, $\alpha$ will be greater than 0 and preferably lies between 0.5 and 0.9. Values of $\alpha$ close to unity will in general be selected for metal on metal or ceramic components. Intermediate values will be chosen for polyethylene cups and lower values for cushion form bearings. The difference $R_1 - R_2$ will normally be selected to lie between 0 and 1mm.

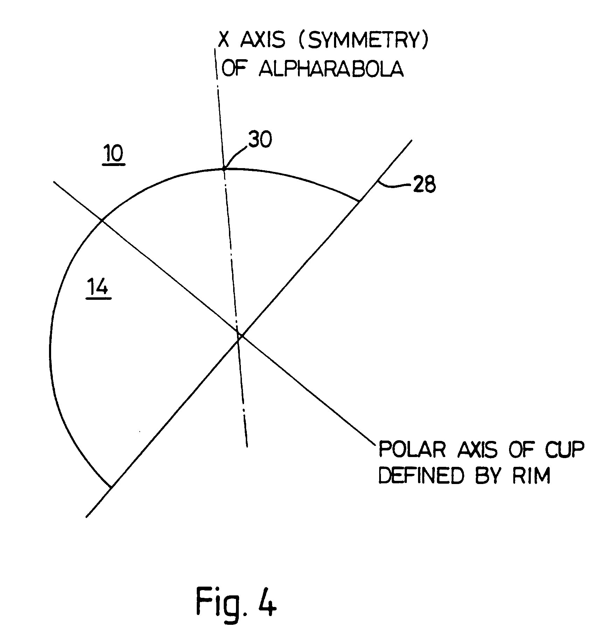

As can be seen from figure 4, the axis of symmetry of the alpharabola need not coincide with the polar axis of the cup, ie. need not be normal to the rim 28 of the cup 10. The point of minimum radius of curvature, referenced 30 will be positioned so that the normal to the surface at that point coincides with the direction at which load on the joint will predominantly be applied.

**Claims**

1. An acetabular cup (10) having a concave bearing socket adapted to receive the head (12) of the femoral component of a hip prosthesis, **characterised in that** the radius of curvature of the surface (14) of the socket continuously and monotonically decreases from the lip of the socket to a minimum $R_2$ at a point within the socket.

2. An acetabular cup (10) according to claim 1 in which the surface of the socket (14) approximates that of a sphere at the said point.

3. An acetabular cup (10) according to claim 1 in which the surface of the socket (14) approximates that a sphere at the intersection of the surface of the socket with a plane.

4. An acetabular cup (10) according to claim 2 or claim 3 in which the surface of the socket (14) is a surface of revolution about the normal to the surface at the said point of minimum radius of curvature.

5. An acetabular cup (10) according to claim 4 in which the surface of revolution is defined by the following relationship:

$$\alpha x^2 + y^2 + z^2 = 2R_2 x$$

$$0 < \alpha < 1$$

where:

x is a Cartesian co-ordinate representing distance from the said point along the said normal; and

y and z are the remaining Cartesian co-ordinates.

6. An acetabular cup (10) according to claim 5 is which:

$$0.5 \leq \alpha \leq 0.9$$

7. A hip prosthesis comprising an acetabular cup (10) according to any preceding claim and a femoral component having a substantially spherical head (12) the concave bearing socket being adapted to receive the head of the femoral component, in which $R_2$ is no greater than the radius of the spherical head of the femoral component $R_1$.

8. A hip prosthesis according to claim 7 in which $R_2$ is equal to $R_1$ and the surface of the socket approximates that of a sphere of radius $R_1$ at the said point.

9. A hip prosthesis according to claim 7 is which $R_2$ is less than $R_1$ and the surface of the socket approximates that of a sphere of radius $R_1$ at the intersection of the surface of the socket with a plane.

10. A hip prosthesis according to claim 9 in which:

$$0 \leq R_1 - R_2 \leq 1mm$$

**Patentansprüche**

1. Hüftgelenkpfanne (10) mit einer konkaven Lagerschale, die dazu ausgestaltet ist, den Kopf (12) des femoralen Teils einer Hüftprothese aufzunehmen, **dadurch gekennzeichnet, dass** der Krümmungsradius der Oberfläche (14) der Schale von dem Öffnungsrand der Schale ausgehend kontinuierlich und monoton abnimmt bis zu einem Minimum $R_2$ an einem Punkt innerhalb der Schale.

2. Hüftgelenkpfanne (10) nach Anspruch 1, bei der die Oberfläche der Schale (14) an dem besagten Punkt diejenige einer Kugel annähert.

3. Hüftgelenkpfanne (10) nach Anspruch 1, bei der die Oberfläche der Schale (14) in dem Schnittbereich der Oberfläche der Schale mit einer Ebene die Oberfläche einer Kugel annähert.

4. Hüftgelenkpfanne (10) nach Anspruch 2 oder 3, bei der die Oberfläche der Schale (14) eine Rotationsfläche um die Normale auf der Oberfläche an dem besagten Punkt mit minimalem Krümmungsradius ist.

5. Hüftgelenkpfanne (10) nach Anspruch 4, bei der die Rotationsfläche durch die folgende Beziehung definiert ist:

$$ax^2 + y^2 + z^2 = 2R_2 X$$

$$0 < a < 1$$

wobei:

x eine Cartesische Koordinate ist, die den Abstand von dem besagten Punkt entlang der Normalen repräsentiert, und

y und z die verbleibenden Cartesischen Koordinaten sind.

6. Hüftgelenkpfanne (10) nach Anspruch 5, bei der:

$$0{,}5 \leq a \leq 0{,}9.$$

7. Hüftprothese mit einer Hüftgelenkpfanne (10) nach einem der vorhergehenden Ansprüche und einem femoralen Teil mit einem im Wesentlichen kugelförmigen Kopf (12), wobei die konkave Lagerschale dazu ausgestaltet ist, den Kopf des femoralen Teils aufzunehmen, wobei $R_2$ nicht größer als der Radius des kugelförmigen Kopfes des fermoralen Teils $R_1$ ist.

8. Hüftprothese nach Anspruch 7, bei der $R_2$ gleich $R_1$ ist und die Oberfläche der Schale an dem besagten Punkt diejenige einer Kugel des Radius $R_1$ annähert.

9. Hüftprothese nach Anspruch 7, bei der $R_2$ kleiner als $R_1$ ist und die Oberfläche der Schale mit dem Radius $R_1$ an dem Schnittbereich der Oberfläche der Schale mit einer Ebene die Oberfläche einer Kugel annähert.

10. Hüftprothese nach Anspruch 9, bei der:

$$0 \leq R_1\text{-}R_2 \leq 1\text{mm}.$$

**Revendications**

1. Cavité cotyloïde (10) possédant un coussinet de support concave adapté pour recevoir la tête (12) du composant fémoral d'une prothèse de hanche, **caractérisé en ce que** le rayon de courbure de la surface (14) du coussinet diminue d'une manière continue et monotone depuis le bord du coussinet jusqu'à une valeur minimale $R_2$ en un point situé à l'intérieur du coussinet.

2. Cavité cotyloïde (10) selon la revendication 1, dans laquelle la surface du coussinet (14) correspond approximativement à celle d'une sphère au niveau dudit point.

3. Cavité cotyloïde (10) selon la revendication 1, dans laquelle la surface du coussinet (14) correspond approximativement à celle d'une sphère au niveau de l'intersection de la surface du coussinet avec un plan.

4. Cavité cotyloïde (10) selon la revendication 2 ou la revendication 3, dans laquelle la surface du coussinet (14) est une surface de révolution autour de la normale à la surface présentant le rayon de courbure minimum.

5. Cavité cotyloïde (10) selon la revendication 4, dans laquelle la surface de révolution est définie par la relation suivante:

$$\alpha x^2 + y^2 + z^2 = 2\,R_2 x$$

$$0 < \alpha < 1$$

avec:

x, coordonnée cartésienne représentant la distance par rapport audit point le long de ladite normale; et
y et z, les autres coordonnées cartésiennes.

6. Cavité cotyloïde (10) selon la revendication 5, dans laquelle:

$$0{,}5 \leq \alpha \leq 0{,}9.$$

7. Prothèse de hanche comprenant une cavité cotyloïde (10) selon l'une quelconque des revendications précédentes, et un composant fémoral possédant une tête sensiblement sphérique (12), le coussinet de support concave étant adapté pour recevoir la tête du composant fémoral, dans laquelle $R_2$ n'est pas supérieur au rayon de la tête sphérique du composant fémoral $R_1$.

8. Prothèse de hanche selon la revendication 7, dans laquelle $R_2$ est égal à $R_1$ et la surface du coussinet correspond approximativement à celle d'une sphère de rayon $R_1$ en ce point.

9. Prothèse de hanche selon la revendication 7, dans laquelle $R_2$ est inférieur à $R_1$ et la surface du coussinet correspond approximativement à celle d'une sphère de rayon $R_1$ au niveau de l'intersection de la surface du coussinet avec un plan.

10. Prothèse de hanche selon la revendication 9, dans laquelle:

$$0 \leq R_1 - R_2 \leq 1\text{ mm}.$$

18

12

10

16

14

20

$R_1 = 9.8$
HEAD

$R_2 = 10$
CUP

X

SPHERE ON SPHERE

Fig.1

22

$R_2 = 10$
CUP
12

X

10

24

16

$R_1 = 10.5$
HEAD SPHERE

14

20

SPHERE ON $\alpha$
CRESCENT

$\alpha = 0.5$

Fig. 2

$R_2=10$
CUP
12

26

10

X

16

$R_2=10$
HEAD

20

14

SPHERE ON $\alpha$
POLAR

$\alpha = 0.5$

Fig. 3

X AXIS (SYMMETRY) OF ALPHARABOLA

30

10

28

14

POLAR AXIS OF CUP DEFINED BY RIM

Fig. 4